# EUROPEAN PATENT SPECIFICATION

(11) **EP 2 566 462 B1**
(45) Date of publication and mention of the grant of the patent: **08.07.2020**
(21) Application number: 10720687.2
(22) Date of filing: 04.05.2010
(51) Int. Cl.: A61K 9/20, A61K 31/19

(54) **IMMEDIATE RELEASE FORMULATIONS AND DOSAGE FORMS OF GAMMA-HYDROXYBUTYRATE**
FORMULIERUNGEN MIT UNMITTELBARER FREISETZUNG UND DARREICHUNGSFORMEN VON GAMMA-HYDROXYBUTYRAT
FORMULATIONS À LIBÉRATION IMMÉDIATE ET FORMES PHARMACEUTIQUES DE GAMMA-HYDROXYBUTYRATE

(43) Date of publication of application: 13.03.2013
(73) Proprietor: Jazz Pharmaceuticals Inc., Palo Alto, CA 94304 (US)
(72) Inventor: MURPHY, Maura Patricia, Baltimore Maryland 21231 (US); PFEIFFER, James Frederick, Oakland California 94611 (US); ALLPHIN, Clark Patrick, Los Altos California 94022 (US); MCGINLAY, Alya Khan, Belmont California 94002 (US); ROURKE, Andrea Marie, Belmont CA 94002 (US)
(74) Representative: Bennett, Nicholas
(86) International application number: PCT/US2010/033572
(87) International publication number: WO 2011/139271

(56) References cited:
- WO-A1-2010/053691
- US-A- 4 983 632
- US-A- 5 594 030
- US-A1- 2006 210 630

## Description

### BACKGROUND OF THE INVENTION

Initial interest in the use of sodium oxybate as a potential treatment for narcolepsy arose from observations made during the use of sodium oxybate (the sodium salt of gamma-hydroxybutyrate) for anesthesia. Unlike traditional hypnotics, sodium oxybate induces sleep that closely resembles normal, physiologic sleep (Mamelak et al., Biol Psych 1977:12:273-288). Therefore, early investigators administered gamma-hydroxybturate (GHB) to patients suffering from disorders of disturbed sleep, including narcolepsy (Broughton et al. in Narcolepsy, NY, NY: Spectrum Publications, Inc. 1976:659-668), where it was found to increase total nocturnal sleep time, decrease nocturnal awakenings and increase Stage 3-4 (slow wave) sleep. Three open-label and two placebo-controlled studies provided a body of evidence demonstrating that improvements in nocturnal sleep were associated with a reduction in cataplexy and improvements in excessive daytime sleepiness (Broughton et al., Can J. Neurol Sci 1979; 6:1-6, and Broughton et al., Can J. Neurol Sci 1980; 7:23-30)

Scharf et al. conducted an open-label study to evaluate the effects of GHB on the sleep patterns and symptoms of non-narcoleptic patients with fibromyalgia (Scharf et al., J Rheumatol 1998;25: 1986-1990). Eleven patients with previously confirmed diagnosis of fibromyalgia who reported at least a 3-month history of widespread musculoskeletal pain in all body quadrants and tenderness in at least five specific trigger point sites participated in the study. Results showed that patients reported significant improvements in the subjective assessments of their levels of pain and fatigue over all 4 weeks of GHB treatment as compared to baseline, as well as a significant improvement in their estimates of overall wellness before and after GHB treatment.

WO 2006/053186 to Frucht describes an open label study of five patients with hyperkinetic movement disorders including ethanol responsive myoclonus and essential tremor. Sodium oxybate was reported to produce dose-dependent improvements in blinded ratings of ethanol responsive myoclonus and tremor and was said to be tolerated at doses that provided clinical benefit.

Xyrem® sodium oxybate oral solution, the FDA approved treatment for cataplexy and excessive daytime sleepiness associated with narcolepsy, contains 500 mg sodium oxybate/ml water, adjusted to pH = 7.5 with malic acid. In man, the plasma half-life of sodium oxybate given orally is about 45 minutes and doses of 2.25 grams to 4.5 grams induce about 2 to 3 hours of sleep (See, L. Borgen et al., J. Clin. Pharmacol., 40, 1053 (2000)). For optimal clinical effectiveness in narcolepsy, sodium oxybate must be given twice during the night, and is administered as an aqueous solution. For each dose, a measured amount of the oral solution must be removed from the primary container and transferred to a separate container where it is diluted with water before administration. The second dose is prepared at bedtime and stored for administration in the middle of the night. This regimen is cumbersome and may be susceptible to errors in the preparation of the individual doses. For this reason, a more convenient unit dosage form of the drug would be clinically advantageous. Sodium oxybate is highly water-soluble, hygroscopic and strongly alkaline. Paradoxically, despite its high water solubility, it can exhibit poor dissolution when formulated in a tablet with common excipients. These properties, along with the large amount of the drug that is required to achieve the clinical effect, present challenges in preparing solid unit dosage forms that are designed for immediate release of the sodium oxybate into the gastrointestinal tract of the user.

The document US2006/210630 A1 discloses immediate release pellets, filled into capsules for oral delivery.

In first and second aspects the invention provides compressed tablet formulations according to claims 1 and 2 respectively.

### BRIEF DESCRIPTIONS OF THE DRAWINGS

Figure 1 is a graph depicting the dissolution profiles of wet and dry-granulated immediate release formulations as disclosed herein.
Figure 2 is a graph showing the dissolution profiles of immediate release formulations as disclosed herein.
Figure 3 graph showing the effect of lubricant on the dissolution profiles of immediate release formulations as disclosed herein.

### DETAILED DESCRIPTION

Formulations and dosage forms for the immediate release of a drug are described herein. Formulations described herein are suited to the immediate release of high dose of sodium oxybate which is a highly water soluble drug. In addition, the formulations described herein provide immediate release of sodium oxybate which is highly hygroscopic, even where said drug must be administered at relatively high doses. The immediate release formulations are provided as a unit dosage form which is a compressed immediate release tablet.

The formulations and unit dosage forms provided herein are utilized to achieve immediate release of sodium salt of GHB (syn. sodium oxybate).

Administration of GHB in solid form presents several challenges. The amount of drug taken by the patient for each dose is high, generally at least 1.5 grams and as high as 4.5 grams. Patients treated with GHB may have difficulty taking solid medications by mouth either because they have disease states that make handling and swallowing difficult or because they must take the medication upon being awakened in the middle of the night. The situation is exacerbated by the large quantity of drug that is administered in each dose. Accordingly, it is desirable to keep the size of the tablet as small as possible while incorporating the largest amount of active ingredient. In addition, if an immediate release tablet is to achieve bioequivalency with the existing Xyrem® oral solution, such a formulation should dissolve quickly without high levels of excipients to speed dissolution.

As used herein, the term "GHB" refers to gamma-hydroxybutyrate. The immediate release GHB compositions described herein comprise a therapeutically effective amount of sodium oxybate. The structure of sodium oxybate is given below as formula (Ia): 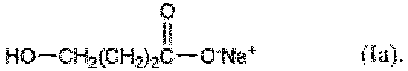 Other salts, which are useful in understanding the invention, include compounds of formula (I): 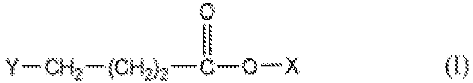 wherein X is a pharmaceutically-acceptable cation and may be selected from the group consisting of potassium, calcium, lithium and magnesium, and Y is OH. Sodium gamma-hydroxybutyrate (sodium oxybate), is currently available from Jazz Pharmaceuticals, Inc. as Xyrem® oral solution.

A "delivery rate" refers to the quantity of drug released *in vivo* from a formulation (tablet or dosage form) as disclosed herein per unit time, e.g., milligrams of a pharmaceutically acceptable salt, hydrate, isomer, tautomer, solvate or complex of GHB per unit time.

"Immediate release" refers to a composition that releases GHB sodium salt substantially completely into the gastrointestinal tract of the user within a period of less than an hour, usually between about 0.1 and about 1 hour and less than about 0.75 hours from ingestion. Such a delivery rate allows the drug to be absorbed by the gastrointestinal tract in a manner that is bioequivalent to an oral solution. Where sodium oxybate is used as the drug and bioequivalence to the existing Xyrem® sodium oxybate oral solution is sought, rapid release of drug from the immediate release formulations described herein is desirable because following delivery of the Xyrem® oral solution, peak plasma concentration of sodium oxybate occurs within an hour. Such rapid absorption will typically occur for an immediate release unit dosage form, such as a tablet, caplet or capsule, if the drug included in such dosage form dissolves in the upper portion the gastrointestinal tract.

A "dissolution rate" refers to the quantity of drug released *in vitro* from a dosage form per unit time into a release medium. *In vitro* dissolution rates in the studies described herein were performed on dosage forms placed in a USP Type II bath containing water which is stirred while maintained at a constant temperature of 37°C. In some examples, aliquots of the dissolution media were injected into a chromatographic system to quantify the amounts of drug dissolved during each testing interval. In other cases, the dissolution was monitored with conductivity measurements using a dip probe.

By "bioavailability" as used herein is intended the estimated area under the curve, or AUC of the active drug in systemic circulation after oral administration with a dosage form as disclosed herein when compared with the AUC of the active drug in systemic circulation after oral administration of Xyrem® sodium oxybate oral solution. The AUC is affected by the extent to which the drug is absorbed in the GI tract. In the case of sodium oxybate, absorption tends to be greatest in the upper GI tract, so in particular embodiments, the immediate release formulations and dosage forms described herein include formulations that dissolve quickly in order to be bioequivalent to the Xyrem® oral solution.

Products are considered to be "bioequivalent" if the relative mean Cₘₐₓ, AUC₍₀₋ₜ₎ and AUC_{(0-∞)} of the test product to reference product is within 80% to 125%.

"Sodium oxybate oral solution" refers to the product currently known as Xyrem®, a solution that contains 500 mg sodium oxybate/ml water, adjusted to pH = 7.5 with malic acid.

The term "AUC₀₋ₜ" means the area under the plasma concentration curve from time 0 to time t.

The term "AUC_{0-∞}" or "AUC_{0-inf}" means the area under the plasma concentration time curve from time 0 to infinity.

"Cₘₐₓ" refers to the maximum plasma concentration of sodium oxybate. The Cₘₐₓ of a 3 gram dose of immediate release tablets is between 10 and 200 µg/mL, often between 20 and 120 µg/mL. Such profiles are especially desirable for diseases such as narcolepsy, cataplexy, movement disorders such as essential tremor and restless leg syndrome, fibromyalgia and chronic fatigue syndrome.

"Tₘₐₓ" refers to the time to maximum plasma concentration for a given drug, which for sodium oxybate is between 0.5 and 2.5 hours, often between 0.5 and 1.5 hours/ "t_{½}" refers to the time to reduce the plasma concentration by 50% during the terminal elimination phase of the drug, which for sodium oxybate is between 0.4 and 0.9 hours, often between 0.5 and 0.7 hours.

The apparent elimination rate constant is "λ_{z}", which for sodium oxybate may be between 0.5 and 2.5 hours⁻¹.

"Oxybate salt" refers to a compound of formula I wherein X is a pharmaceutically-acceptable cation and may be selected from the group consisting of sodium, potassium, calcium, lithium and magnesium and Y is OH.

"Sodium oxybate" refers to a compound of formula Ia.

Immediate release formulations suitable for oral administration are compressed tablets, which can deliver a therapeutically effective dose of GHB upon ingestion thereof by the patient of one or more of said dosage forms, each of which can provide a dosage of, for example, about 0.5-1.5 g of GHB. Additionally, the immediate release dosage forms can be shaped or scored to facilitate swallowing.

The formulation and structure of an immediate release dosage form as disclosed herein provides immediate release performance that suits a particular dosing need. The immediate release dosage form as disclosed herein provides rapid onset of action, releasing more than about 90%, such as, for example, more than about 95%, of the drug contained therein within a period of time selected from less than one hour, less than 45 minutes, less than 30 minutes and less than 15 minutes after administration.

Moreover, the rate of drug release from an immediate release dosage form as disclosed herein facilitates a desired dosing regimen or achieves targeted dosing. In one embodiment, the immediate release dosage form is formulated to deliver as much as 2,000 mg of GHB sodium salt. In particular embodiments, the total amount of drug contained within an immediate release dosage form according to the present description may be between about 500 mg and about 1,400 mg. For example, in certain such embodiments, the total amount of drug may be selected from between about 500 mg and 1,400 mg, 500 mg and 1,200 mg, 500 mg and 1,100 mg, 600 mg and 1,200 mg, 600 mg and 1,100 mg, 600 mg and 1,000 mg, 600 mg and 950 mg, 600 mg and 850 mg, 600 mg and 750 mg, 750 mg and 1,200 mg, 750 mg and 1,100 mg, 750 mg and 1,000 mg, 750 mg and 950 mg, and 750 mg and 850 mg.

Immediate release dosage forms described herein include immediate release formulations that facilitate high loading of GHB. In certain embodiments, an immediate release formulation as disclosed herein may comprise GHB sodium salt in an amount selected from 95%, 96%, 97%, and 98% by weight of the immediate release formulation. In certain such embodiments, the amount of GHB sodium salt in the immediate release formulation may range from 95-98%, 95-97%, and 95-96.5% by weight of the immediate release formulation. In particular embodiments, even with the high drug loading described herein, the immediate release formulations disclosed herein facilitate production of solid unit dosage forms that are bioequivalent to the Xyrem® sodium oxybate oral solution. In certain such embodiments, the solid unit dosage forms described herein release more than about 95% of the GHB sodium salt contained therein within a period of less than one hour after administration.

The immediate release formulations provided herein generally include GHB sodium salt and some level of lubricant to facilitate processing of the formulations into a unit dosage form. In embodiments of the invention, the immediate release formulations described herein include a combination of GHB sodium salt, lubricant, and binder, as described herein, and in certain such embodiments, the immediate release formulations are substantially free of other excipients or adjuvants. In still further embodiments, the formulations described herein include a combination of GHB, lubricant, binder, and surfactant, as described herein, and in certain such embodiments, the immediate release formulations are substantially free of other excipients or adjuvants. Though the immediate release formulations described herein may be formulated using a combination of drug and one or more of a lubricant, binder and surfactant, in certain embodiments, the compositions described herein may include one or more additional excipients selected from, for example, fillers, compression aids, diluents, disintegrants, colorants, buffering agents, coatings, glidants, or other suitable excipients.

To facilitate processing of the immediate release formulations described herein into unit dosage forms, the immediate release formulations include some level of lubricant. The immediate release formulation includes at least one lubricant selected from magnesium stearate, stearic acid, and sodium stearyl fumarate. One or more lubricants is present in the immediate release formulation, and in some embodiments the total lubricant content is selected from 1%, 1.5% and 2% by weight.

In one embodiment, magnesium stearate may be used in combination with one or more other lubricants or a surfactant, such as sodium lauryl sulfate. In particular, if needed to overcome potential hydrophobic properties of magnesium stearate, sodium lauryl sulfate may also be included when using magnesium stearate (Remington: the Science and Practice of Pharmacy, 20th edition, Gennaro, Ed., Lippincott Williams & Wilkins (2000)).

The immediate release formulations described herein include one or more binders. Binders suitable for use in the immediate release formulations of the present description may be selected from hydroxypropyl cellulose (HPC), hydroxypropyl methylcellulose (HPMC), polyvinyl alcohol, povidone, starch, and sodium carboxymethylcellulose. The immediate release formulation may include a binder in an amount selected from 1%, 1.5% and 2% by weight.
In yet another embodiment, the immediate release formulation includes between 96-98% by weight GHB sodium salt, between 1-2% by weight of a lubricant selected from magnesium stearate, stearic acid, sodium stearyl fumarate, and combinations thereof, and between 1-2% by weight of a binder selected from HPMC and povidone. In the formulations of the immediate release formulations incorporating a binder described herein, the GHB included in such formulations is sodium oxybate.

The immediate release formulation may also include one or more surfactants. For instance, one or more surfactants may be added to formulations that may include poorly soluble excipients in order to facilitate dissolution of these excipients and, indirectly, of the drug. The addition of small amounts of surfactant to the immediate release formulations as disclosed herein may produce an increased dissolution rate. In certain embodiments, the immediate release formulation may include GHB sodium salt in combination with one or more surfactants selected from, for example, ionic and non-ionic surfactants. In one such embodiment, the immediate release formulation may include at least one anionic surfactant, including docusate sodium (dioctyl sulfosuccinate sodium salt) and sodium lauryl sulfate. In yet another embodiment, the immediate release formulation may include at least one non-ionic surfactant selected from polyoxyethyelene alkyl ethers, polyoxyethylene stearates, poloxamers (e.g., poloxamer 188), polysorbate (e.g., polysorbate 80), sorbitan esters, and glyceryl monooleate. In specific embodiments, one or more surfactants included in an immediate release formulation as disclosed herein may be present, for example, in an amount of between about 0.25-2.5% by weight of the immediate release formulation. In other embodiments, one or more surfactants included in an immediate release formulation as disclosed herein may be present in an amount of up to about 3.0% by weight of the immediate release formulation. For example, in certain embodiments, the immediate release formulation may include one or more surfactants present in a range selected from about 0.01% to 3%, 0.01% to 2%, 0.01% to 1%, 0.5% to 3%, 0.5% to 2%, and 0.5% to 1% by weight of the immediate release formulation. In one such embodiment, the immediate release formulation may include about 1% by weight of a surfactant selected from polysorbate 80, poloxamer 188, sodium lauryl sulfate, and docusate sodium.

The immediate release formulation may include about 95-97.5% by weight GHB sodium salt, about 0.5-1% by weight surfactant selected from polysorbate 80, poloxamer 188, sodium lauryl sulfate, and docusate sodium, about 1-2% by weight binder selected from HPMC and povidone, and about 1-2% by weight lubricant selected from magnesium stearate, stearic acid, sodium stearyl fumarate, and combinations thereof.

The immediate release formulations described herein may be manufactured using standard techniques, such as wet granulation, roller compaction, fluid bed granulation, and dry powder blending. Suitable methods for the manufacture of the immediate release formulations and unit dosage forms described herein are provided, for example, in Remington, 20th edition, Chapter 45 (Oral Solid Dosage Forms). It has been found that, even without the aid of binders or non-lubricating excipients, such as compression aids, wet granulation techniques can afford flowable granules with compression characteristics suitable for forming unit dosage forms as described herein. Therefore, in certain formulations, where a drug content greater than about 85%, 90% or 95% by weight is desired for the immediate release formulation, wet granulation techniques may be used to prepare immediate release formulations as described herein. In such formulations, as illustrated in the Examples provided herein, conventional organic or aqueous solvents may be used in the wet granulation process. Suitable wet granulation processes can be performed as fluidized bed, high shear, or low shear (wet massing) granulation techniques, as are known in the art.

In addition to one or more of a GHB drug, lubricant, binder and surfactant, where desired, the immediate release formulations described herein may also include fillers or compression aids selected from at least one of lactose, calcium carbonate, calcium sulfate, compressible sugars, dextrates, dextrin, dextrose, kaolin, magnesium carbonate, magnesium oxide, maltodextrin, mannitol, microcrystalline cellulose, powdered cellulose, and sucrose. Where a filler or compression aid is used, in certain embodiments, it may be included in the immediate release formulation in an amount ranging from about 1%-2.5% by weight. In further such embodiments, the immediate release formulations include about 2.5% by weight microcrystalline cellulose.

Immediate release formulations as described herein may be processed into compressed tablets using conventional techniques. Immediate release dosage forms prepared as described are adapted for oral administration, so as to attain and maintain a therapeutic level of GHB over a preselected interval. In certain embodiments, an immediate release dosage form as described herein may comprise a solid oral dosage form of any desired shape and size including round, oval, oblong cylindrical, or polygonal. In one such embodiment, the surfaces of the immediate release dosage form may be flat, round, concave, or convex.

In particular, the immediate release tablets contain a relatively large percentage and absolute amount of GHB and so are expected to improve patient compliance and convenience, by replacing the need to ingest large amounts of liquids or liquid/solid suspensions. One or more immediate release tablets as described herein can be administered, by oral ingestion, e.g., closely spaced, in order to provide a therapeutically effective dose of GHB to the subject in a relatively short period of time. For example, disintegration of a 500 mg - 1.0 g tablet prepared according to the present description can provide about 80-100% of the GHB to the subject in about 30-60 minutes.

Where desired or necessary, the outer surface of an immediate release dosage form as disclosed herein may be coated with a moisture barrier layer using materials and methods known in the art. For example, where the GHB delivered by the unit dosage form is highly hygroscopic, such as sodium oxybate, providing a moisture barrier layer over the immediate release dosage form as disclosed herein may be desirable. For example, protection of an immediate release dosage form as disclosed herein from water during storage may be provided or enhanced by coating the tablet with a coating of a substantially water soluble or insoluble polymer. Useful water-insoluble or water-resistant coating polymers include ethyl cellulose and polyvinyl acetates. Further water-insoluble or water resistant coating polymers include polyacrylates, polymethacrylates or the like. Suitable water-soluble polymers include polyvinyl alcohol and HPMC. Further suitable water-soluble polymers include PVP, HPC, HPEC, PEG, HEC and the like.

In third and fourth aspects of the invention compressed tablet immediate release formulations for use according to claims 14 and 15 are also provided.

Any references in the description to treatments or to methods of treatment refer to the formulations of the present invention for use in a method of treatment.

Methods are disclosed herein to treat conditions amenable to treatment by GHB, by administering an effective amount of one or more dosage forms as described herein. For example, the present dosage forms can be administered to treat a human afflicted with narcolepsy to reduce cataplexy and/or daytime sleepiness. Furthermore, the dosage forms disclosed herein may be useful in the treatment of a variety of conditions amenable to treatment by GHB, such as to improve the quality of sleep, or in conditions in which an increase in growth hormone levels *in vivo* is desired, and to treat fibromyalgia or chronic fatigue syndrome. See, U.S. Patent No. 5,990,162. The present dosage forms may be used to treat a host of other indications including drug and alcohol abuse, anxiety, cerebrovascular diseases, central nervous system disorders, neurological disorders including Parkinson's Disease and Alzheimer Disease, Multiple Sclerosis, autism, depression, inflammatory disorders, including those of the bowel, such as irritable bowel disorder, regional illitis and ulcerative colitis, autoimmune inflammatory disorders, certain endocrine disturbances and diabetes.

The present dosage forms may also be administered for the purpose of tissue protection including protection following hypoxia/anoxia such as in stroke, organ transplantation, organ preservation, myocardial infarction or ischemia, reperfusion injury, protection following chemotherapy, radiation, progeria, or an increased level of intracranial pressure, e.g. due to head trauma. The present dosage forms can also be used to treat other pathologies believed to be caused or exacerbated by lipid peroxidation and/or free radicals, such as pathologies associated with oxidative stress, including normal aging. See, U.S. Patent Publication US 2004/009245 5 A1. The present dosage forms may also be used to treat movement disorders including restless leg syndrome, myoclonus, dystonia and/or essential tremor. See, Frucht et al, Movement Disorders, 20(10), 1330 (2005).

The dosage forms disclosed herein can also be provided as a kit comprising, separately packaged, a container comprising a plurality of immediate release tablets, which tablets can be individually packaged, as in foil envelopes or in a blister pack. The tablets can be packaged in many conformations with or without dessicants or other materials to prevent ingress of water. Instruction materials or means, such as printed labeling, can also be included for their administration, e.g., sequentially over a preselected time period and/or at preselected intervals, to yield the desired levels of sodium oxybate *in vivo* for preselected periods of time, to treat a preselected condition.

A daily dose of about 1-400 mg/kg of sodium oxybate or other oxybate salt such as a compound of formula (I) can be administered to accomplish the therapeutic results disclosed herein. For example, a daily dosage of about 0.5-20 g of the sodium oxybate or of a compound of formula (I) can be administered, preferably about 3-9 g, in single or divided doses. For example, useful dosages and modes of administration are disclosed in U.S. Pat. Nos. 5,990,162 and 6,472,432. Methods to extrapolate from dosages found to be effective in laboratory animals such as mice, to doses effective in humans are known to the art. See, U.S. Pat. No. 5,294,430, or 4,939,949.

### Examples

### Reference Example 1. Immediate Release Sodium Oxvbate Tablets

This example compares two formulations of compressed tablets of sodium oxybate which have greater than 70% drug loading, one for which granulation was made with wet granulation and the other made by roller compaction. The composition of the tablets is summarized on Table 1, along with quantities to produce batches of 3000 tablets each.

**Table 1**

| Formulation A (wet granulated) | | | |
|---|---|---|---|
| **Ingredient(s)** | **% (w/w)** | **Qty/Unit (mg)** | **Batch Quantity, g** |
| Sodium Oxybate | 71.4 | 750.0 | 2250.0 |
| Microcrystalline Cellulose (Avicel PH 101) | 12.1 | 126.7 | 380.1 |
| Povidone (PVP K-17) | 2.0 | 21.0 | 63.0 |
| Croscarmellose Sodium NF/EP | 12.0 | 126.0 | 378.0 |
| Colloidal Silicon Dioxide (Cab-O-Sil MP5) | 0.50 | 5.3 | 15.9 |
| Sodium Lauryl Sulfate | 1.0 | 10.5 | 31.5 |
| Magnesium Stearate, NF (vegetable grade) | 1.0 | 10.5 | 31.5 |

| Formulation B (dry granulated) | | | |
|---|---|---|---|
| **Ingredient(s)** | **% (w/w)** | **Qty/Unit (mg)** | **Batch Quantity, g** |
| Sodium Oxybate | 78.9 | 750.0 | 2250.0 |
| Microcrystalline Cellulose (Avicel PH 101) | 5.9 | 55.6 | 166.8 |
| Povidone (PVP K-17) | 2.0 | 19.0 | 57.0 |
| Pregelatinized Starch (Starch 1500) | 5.0 | 47.5 | 142.5 |
| Colloidal Silicon Dioxide (Cab-O-Sil MP5) | 0.5 | 4.8 | 14.4 |
| Magnesium Stearate, NF (vegetable grade) (0.7% intragranular, 0.5% extragranular) | 1.2 | 11.4 | 34.2 |
| Croscarmellose Sodium, NF/EP (Ac-Di-Sol SD-711) (4% intragranular, 2.5% extragranular) | 6.5 | 61.8 | 185.4 |

Formulation A was produced by wet granulation in a planetary mixer. The sodium oxybate, microcrystalline cellulose, povidone, half of the sodium lauryl sulfate, and 58% of the croscarmellose sodium were pre-blended dry. The remainder of the sodium lauryl sulfate dissolved in the water used to granulate. The amount of water added was 8% of the dry powder weight. The material was mixed until uniform granules were made, then wet-sized through a #6 mesh screen, followed by oven drying at 60C so that a final moisture content (loss on drying) was between 1.0% and 2.5%. The dried granulation was then milled through a #14 screen using a Comil. Finally, the remainder of the croscarmellose sodium was blended into the milled granulation with an 8-quart V-blender for 5 minutes, and the magnesium stearate was then added and blended for an additional 3 minutes.

To prepare Formulation B by roller compaction, first all the ingredients were hand-screened through a 20 mesh screen. All of the ingredients except the magnesium stearate and 43% of the croscarmellose sodium were transferred to an 8-quart V blender, and mixed for five minutes. The intragranular portion of the croscarmellose sodium was blended in the V-blender for 5 minutes, and finally the intragranular portion of the magnesium stearate (20.0 g) was added to the blender and mixing continued for 3 minutes.. The blended powder was passed through a Vector TF-156 roller compactor set to a target pressure of 47 kg/cm², roller speed and screw speed both at 4RPM. Ribbons with thickness of 1.4 ± 0.05 mm were made without added water. The ribbons were granulated using an in-line rotary mill fitted with a 16-mesh screen. The granulate was added to the blender and mixed for 5 minutes. The remaining magnesium stearate (14.2 g) and croscarmellose sodium (71.4 g) was added to the blend, and mixed for 3 minutes.

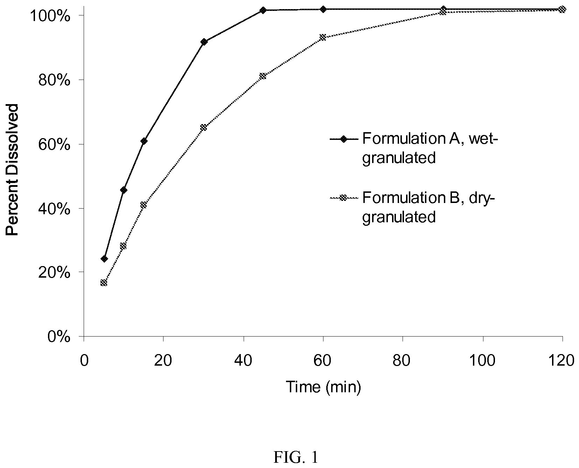

The two granulations were compressed into tablets on a 15-station standard rotary press fitted with 0.3366" x 0.7283" oblong tooling. The target weights for A and B were 1050 mg and 950 mg, respectively, to achieve a target potency of 750 mg/tablet. The dissolution profiles, shown in FIG. 1, demonstrate more than 90% is dissolved in 60 minutes.

### Reference Example 2. Bioavailability and Bioequivalence of Sodium Oxybate Tablets

The formulations of Reference Example 1 were tested for bioequivalence to sodium oxybate oral solution (Xyrem®). A Phase I, three-way, open-label, randomized single-dose crossover study of Formulation A (4.5 grams of Formulation A given as 6 tablets: Treatment A), Formulation B (4.5 grams of Formulation B given as 6 tablets: Treatment B), and Xyrem® (4.5 grams of sodium oxybate oral solution: Treatment C). Following a 1 to 21-day screening period, the study duration for each subject was approximately 7 days, Period 1 comprising Days 1 to 2, Period 2 comprising Days 3 to 4, and Period 3 Days 5 to 6. A 2-day washout period (dosing on the morning of the first day followed by a 1 day washout) separated the Treatments A, B and C.

Single doses (4.5 g, given as 6 x 750 mg tablets) of sodium oxybate solid dosage Formulations A and B and Single doses (4.5 g) of sodium oxybate oral solution (Xyrem®) were administered orally in the morning following a 10-hour fast, with subjects remaining fasted for a further 4 hours after dosing. The PK profile for sodium oxybate was evaluated over an 8-hour period, based on blood samples (5 mL) collected pre-dose; at 10, 20, 30, 45, 60 and 75 minutes post-dose; and at 1.5, 2, 2.5, 3, 3.5, 4, 4.5, 5, 6, 7 and 8 hours post-dose following each treatment. The PK parameters calculated for plasma sodium oxybate concentrations included: the area under the plasma concentration time curve from time 0 to time t of the last quantifiable concentration [AUC₀₋ₜ], and area under the plasma concentration time curve from time 0 to infinity[AUC_{0-∞}], maximum plasma concentration of sodium oxybate (Cₘₐₓ), time to maximum plasma concentration (tmax), the apparent elimination rate constant (λ_{z}) and half-life (t_{½}) and the relative bioavailability for solid dosage Formulations A and B versus Xyrem®.

The relative bioavailability of Treatments A and B versus Treatment C (Xyrem®) based on AUC values were 98% and 100%, respectively. All treatments were found to be bioequivalent with regard to Cₘₐₓ and total exposure AUC after oral administration of sodium oxybate. Since no tablet formulation can dissolve faster than Xyrem® liquid, this study suggests that any tablet formulation dissolving at least 80% in 45 minutes should be bioequivalent to Xyrem®.

**Table 2**

| Summary of Mean (SD) Sodium Oxybate Pharmacokinetic Parameters | | | | | |
|---|---|---|---|---|---|
| PK Parameter | Units | | Treatment A (Test | Treatment B (Test) | Treatment C (Reference) |
| Cₘₐₓ | (µg/mL) | Mean | 129 | 135 | 143 |
| | | SD | 37.6 | 37.2 | 29.2 |
| | | Geometric Mean | 123 | 131 | 140 |
| | | Geometric SD | 1.39 | 1.32 | 1.23 |
| tₘₐₓ | (hr) | Median | 1.00 | 1.00 | 0.750 |
| | | Min, Max | 0.750, 2.50 | 0.500, 2.50 | 0.500, 1.50 |
| AUC₀₋ₜ | (µg*hr/mL) | Mean | 297 | 303 | 298 |
| | | SD | 104 | 112 | 96.1 |
| | | Geometric Mean | 275 | 281 | 281 |
| | | Geometric SD | 1.53 | 1.53 | 1.45 |
| AUC_{0-inf} | (g*hr/mL) | Mean | 298 | 305 | 300 |
| | | SD | 104 | 112 | 96.4 |
| | | Geometric Mean | 277 | 282 | 283 |
| | | Geometric SD | 1.53 | 1.53 | 1.45 |
| t_{1/2} | (hr) | Mean | 0.584 | 0.556 | 0.644 |
| | | SD | 0.196 | 0.128 | 0.245 |
| λ_{z} | (hr⁻¹) | Mean | 1.29 | 1.33 | 1.19 |
| | | SD | 0.414 | 0.398 | 0.345 |

### Reference Example 3 - Dry-granulated formulation

The roller-compaction method of granulation was chosen for further development and formulation optimization to maximize drug loading while producing tablets of acceptable quality. Two changes - addition of sodium lauryl sulfate as surfactant and removal of croscarmellose sodium - resulted in Formulation C, which has 84.2% drug loading and was successfully processed using scaled-up techniques for roller compaction described in Reference Example 1. Table 1 shows the formulation and batch quantities to produce about 120,000 tablets.

The dry powders, except for magnesium stearate, were passed through a Fitzmill set at low speed with knives forward), then charged to a 10 cu-ft. V-blender and mixed for 130 seconds (39 revolutions). The intragranular magnesium stearate (0.534 kg) was passed through a 20-mesh screen and then added to the V-blender containing the other powders and blended for 77 seconds (23 revolutions). Roller compaction was performed on a Fitzpatrick Chilsonator (TG 99) with axially grooved rollers (1 ½" wide and 8" diameter) set at 8 rpm roller speed, 25 rpm horizontal screw feed, 200 rpm vertical screw speed, about 22 psi booster pressure, 750 psi roller pressure, and 6°C chiller temperature. The material was screened through a 30" Sweeco equipped with a 14-mesh screen. About 16% of the "fines" material was passed through the chilsonator a second time. The collected product was milled through a Fitzmill, and a 100g sample was analyzed for sieve fractions. The amount retained on 20 mesh, 40, 60, 80, 120, 200, 325-mesh, and in the pan was, respectively, 17.7.0%, 16.1%, 13.1%, 8.3%, 10.4%, 10.3%, 9.0%, and 14.1%.

**To** 104.2 kg of the collected granulation, 1.05 kg of magnesium stearate was added and mixed in a V-blender for 77 seconds (23 revolutions). The blended granulation was then compressed on a D-tooled Hata tablet press with 26 sets of 0.3290" x 0.7120" oblong tooling. Parameters were adjusted to yield 891 mg tablet weight, 5.8-5.9 mm thickness, 9.1-13 kP hardness, and about 0.02% friability. 95.7 kg of acceptable tablets were produced.

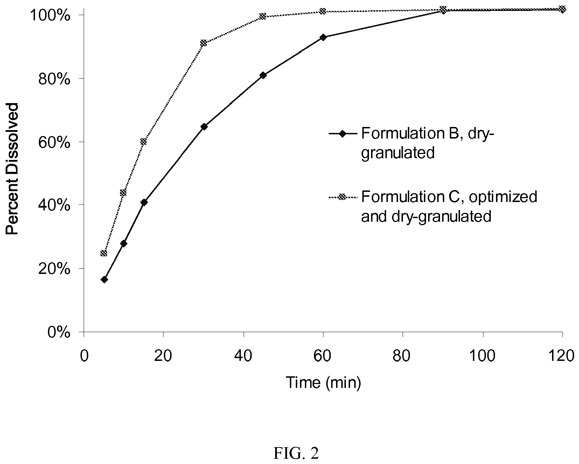

The dissolution profile, shown in FIG. 2, demonstrates substantially faster dissolution than that observed with the original dry-granulated product of Formulation B.

**Table 3**

| Dry-Granulated Formulation C | | | |
|---|---|---|---|
| **Ingredient(s)** | **% (w/w)** | **Qty/Unit (mg)** | **Batch Quantity (kg)** |
| Sodium Oxybate | 84.17 | 750.0 | 90.00 |
| Microcrystalline Cellulose (Avicel PH 101) | 5.83 | 51.9 | 6.23 |
| Povidone (PVP K-17) | 2.00 | 17.8 | 2.14 |
| Pregelatinized Starch (Starch 1500) | 5.00 | 44.4 | 5.34 |
| Colloidal Silicon Dioxide (Cab-O-Sil MP5) | 0.50 | 4.4 | 0.53 |
| Sodium Lauryl Sulfate | 1.00 | 8.9 | 1.07 |
| Magnesium Stearate, NF (vegetable grade) (0.5% intragranular, 1.0% extragranular) | 1.50 | 13.35 | 1.60 |

### Example 4- Higher drug-loaded formulation for wet granulation

The formulation consisted of a low level of binder, a lubricant, and the sodium oxybate. The granulation was manufactured in a TK Fielder 25 L high shear granulator according to the formula in Table 1A. The binder, hydroxypropyl cellulose (Klucel EXF), was divided into two equal portions; half was dissolved in the ethanol, and half was dry blended with sodium oxybate. The material was initially granulated with 10% w/w ethanol and then titrated with another 3.5% w/w ethanol solution to achieve desired granule growth. A suitable wet mass was obtained at a total ethanol concentration of 13.5% w/w. The wet granules were divided into two sublots and then each sublot was dried in a 5-liter Niro fluid bed dryer. The dried granules were combined and milled through a Comil® equipped with a 14 mesh screen. The granulation was then blended with 2% magnesium stearate lubricant. Granulation parameters and particle size distribution are shown in Tables 4B and 4C, respectively.

**Table 4A**

| Immediate-Release Tablet Formulation | | | |
|---|---|---|---|
| | Ingredient(s) | % w/w | mg/tablet |
| 1 | Sodium Oxybate | 96.0 | 750.0 |
| 2 | Hydroxypropyl cellulose, NF (Klucel EXF) | 2.0 | 15.6 |
| 3 | Ethanol, USP (200 proof)* | *13.5* | |
| 4 | Magnesium Stearate, NF | 2.0 | 15.6 |
| | TOTAL | 100.0 | 781.2 |

| | | | |
|---|---|---|---|
| * Granulation solvent, removed during drying step | | | |

**Table 4B**

| Granulation Parameters | | |
|---|---|---|
| **Wet granulation** | | |
| Granulation solution addition rate (g/min) | 250 | |
| Total granulation time (including solution addition and wet massing time) | 7 minutes | |
| Impeller speed (rpm) | 300 | |
| Chopper speed (rpm) | 1800 | |

| **Drying** | Sublot 1 | Sublot 2 |
|---|---|---|
| Drying inlet temperature (°C) | 70 | 70 |
| Total drying time (min) | 17 | 18 |
| Exhaust temperature at end of drying (°C) | 47 | 48 |
| LOD (% wt loss) | 0.84 | 0.92 |

**Table 4C**

| Screen Analysis of Milled Granulation | | |
|---|---|---|
| Screen size US Std mesh | Opening size microns | Wt Retained (%) |
| 20 | 850 | 2.1 |
| 40 | 420 | 10.4 |
| 60 | 250 | 19.8 |
| 80 | 180 | 25.0 |
| 120 | 125 | 22.9 |
| 200 | 75 | 12.5 |
| Pan | <45 | 7.3 |

### Example 5 - Effect of Tablet Shape

The formulation of Example 4, containing 96% sodium oxybate, 2% HPC ExF, and 2% magnesium stearate, was produced in two batches using the procedures described in Example 4. One batch was compressed on a rotary press with 0.3266" x 0.7283" oblong (capsule-shaped) tooling, whereas the other batch was compressed with 0.325" x 0.705" modified oval tooling. In both cases, acceptable hardnesses (>10 kiloponds) and low friability were achieved. The dissolution behavior, as shown in Table 5, indicates that the oblong shape afforded substantially faster dissolution. This is likely due to a combination of a flatter surface, thinner tablet, and higher surface area.

**Table 5**

| Comparison of Tablet Shape | | | | | | |
|---|---|---|---|---|---|---|
| Tablet | % Dissolved vs. Time (minutes) | | | | Minutes to dissolve: | |
| Shape | 5 | 15 | 30 | 45 | 50% | 80% |
| 0.325" x 0.705" Oval | 24% | 42% | 58% | 68% | 22.8 | 67.2 |
| 0.3266" x 0.7283" Oblong | 26% | 47% | 65% | 78% | 17.3 | 48.6 |

### Example 6- Effect of Binder Type and Solvent

Several binders were evaluated using either water or denatured alcohol as solvent. For the water-based binders, solutions or gels of 20% binder were prepared as 1.25 grams binder added to 5.0 grams water. These aqueous preparations were vigorously mixed and stored at 60°C until used.

For the alcohol-based granulations, about 1.0 grams of binder solution (10% binder in denatured alcohol) was added to 5.0 grams sodium oxybate while stirring vigorously for about 1 minute. For the water-based granulations, about 0.5 grams of gel or solution was weighed into a beaker. A10-fold amount of sodium oxybate was added to this, and then vigorously stirred for 1-3 minutes until granules formed. The granulations were wet sieved through a 16-mesh screen, dried at 60°C for about 1 hour, and then dry sieved through a 16-mesh screen prior to blending required amount to obtain a 2% magnesium stearate level. For the water-based granulations, continued overnight drying (open container at 60°C) was required.

For each granulation, four tablets of 781 mg were compressed using 0.3266" x 0.7283" oblong tooling and a Carver press operated at 1-ton force and about 4-seconds dwell time. Two of the tablets were tested for hardness. The other two were tested for dissolution by USP Apparatus 2 in 900 ml of de-ionized water, with paddles rotating at 50 rpm, and two tablets dropped in each vessel. The results shown in Table 6 suggest that either water or alcohol is suitable solvent povidone and hydroxypropyl cellulose, that a variety of conventional binders are suitable for producing granulations of appropriate size and flowability, and that these granulations generally produce tablets of sufficient hardness. Furthermore, use of a binder may be optional in some circumstances.

**Table 6**

| Binder Screening | | | | | | | | |
|---|---|---|---|---|---|---|---|---|
| Formulations contain only binder, sodium oxybate, and 2% magnesium stearate | | | | | | | | |
| Binder | Solvent | Tablet Hardness (N) | % Dissolved vs. Time (minutes) | | | | Minutes to dissolve: | |
| | | | 5 | 15 | 30 | 45 | 50% | 80% |
| HPMC E5 | Water | 58 | 26% | 51% | 72% | 86% | 14.4 | 37.9 |
| HPC EF | Water | 73 | 25% | 51% | 70% | 84% | 14.7 | 40.8 |
| NaCMC 7L | Water | 73 | 24% | 49% | 68% | 82% | 15.9 | 43.1 |
| PVA | Water | 80 | 28% | 54% | 75% | 90% | 12.8 | 34.8 |
| PVP K30 | Water | 108 | 36% | 62% | 89% | 99% | 9.1 | 23.2 |
| Starch 1500 | Water | 103 | 22% | 44% | 62% | 74% | 19.2 | 55.9 |
| PVP K30 | Alcohol | 102 | 27% | 54% | 76% | 91% | 12.9 | 33.3 |
| HPC EF | Alcohol | 103 | 22% | 46% | 63% | 75% | 18.2 | 48.2 |
| No binder | Alcohol | 74 | 30% | 61% | 81% | 95% | 10.1 | 28.8 |

| | | | | | | | | |
|---|---|---|---|---|---|---|---|---|
| Binders (suppliers) in order: Hypromellose (Dow), hydroxypropyl cellulose (Ashland), sodium carboxymethyl cellulose (Ashland), polyvinyl alcohol, povidone (BASF), pregelatinized maize starch (Colorcon) | | | | | | | | |

### Example 7 -- Effect of lubricant level

A binder solution of 10% povidone (PVP K30) was prepared by dissolving 4.0 grams of PVP K30 (BASF) in 36.1 grams of denatured alcohol. To 19.48 grams of sodium oxybate powder, 4.00 grams of binder solution was added while mixing by hand in a beaker. The wet mass was sieved through a 16-mesh screen, dried at 60°C for about 1 hour, and then sieved through a 16-mesh screen to yield 18.61 grams of granulation. The granulation was divided into 2.5 gram aliquots, and to each aliquot was added the required amount of magnesium stearate to make 0%, 0.5%, 1%, 1.5%, 2.0%, and 2.5% of the granulation. The lubricant was blended for approximately 30 seconds by rotating and inverting the closed container about 30 cycles.

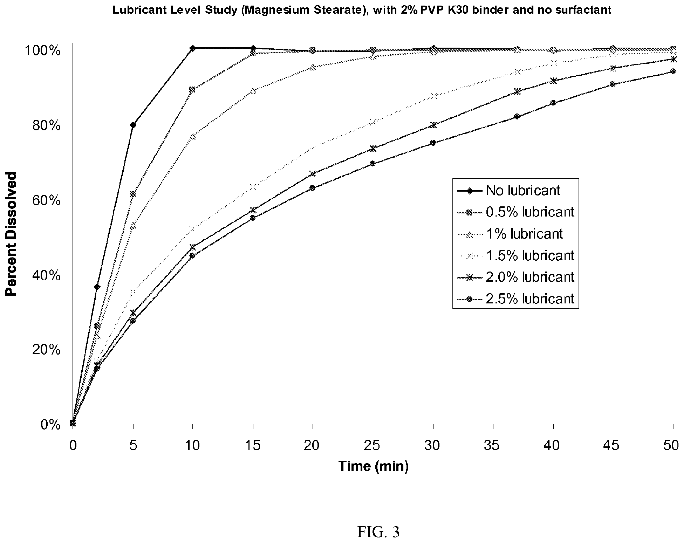

The blends were compressed into 2 tablets each of 783 mg using 0.3266" x 0.7283" oblong tooling and a Carver press operated at 1-ton force and about 4-seconds dwell time. The compressed tablets were tested for dissolution by USP Apparatus 2 in 900 ml of de-ionized water, with paddles rotating at 50 rpm, and two tablets dropped in each vessel. Assay by conductivity (dip probe) was performed at 2 minutes and then at about every 5 minutes until 50 minutes. The results are represented in Table 7 and FIG. 3.

**Table 7**

| Effect of Magnesium Stearate Level | | | | | | |
|---|---|---|---|---|---|---|
| Magnesium Stearate level | % Dissolved vs. Time (minutes) | | | | Minutes to dissolve: | |
| | 5 | 15 | 30 | 45 | 50% | 80% |
| 0.0% | 80% | 101% | 100% | 101% | 2.9 | 5.0 |
| 0.5% | 62% | 99% | 100% | 100% | 4.0 | 8.3 |
| 1.0% | 53% | 89% | 100% | 100% | 4.7 | 11.2 |
| 1.5% | 35% | 63% | 88% | 99% | 9.4 | 24.5 |
| 2.0% | 30% | 57% | 80% | 95% | 11.3 | 29.9 |
| 2.5% | 28% | 55% | 75% | 91% | 12.5 | 34.9 |

### Example 8- Surfactant Screening

Several surfactants were screened for effectiveness at reducing the dissolution time of tablets. A master binder solution of 10% PVP K30 was prepared by dissolving 4.00 grams of PVP K30 in 36.1 grams of denatured alcohol. Each of the surfactants was applied in solution with the binder by adding about 0.15 grams of surfactant to 3.00 grams of the binder solution. In each case, about 4.8 grams of sodium oxybate was mixed with about 1.0 grams of surfactant-containing binder solution to form granules which were then sieved through a 16-mesh screen. After drying about 1 hour, the granulations were sieved dry through a 16-mesh screen, and compressed into two tablets each of 783 mg using 0.3266" x 0.7283" oblong tooling and a Carver press operated at 1-ton force and about 4-seconds dwell time. The tablets were tested for dissolution by USP Apparatus 2 in 900 ml of de-ionized water, with paddles rotating at 50 rpm, and two tablets dropped in each vessel. Assay by conductivity (dip probe) was performed at 2 minutes and then at about every 5 minutes until 45 minutes.

The results shown are shown in Table 8.

**Table 8**

| Effect of Surfactant Type | | | | | | |
|---|---|---|---|---|---|---|
| Formulations containing 2% PVP K30, 95% sodium oxybate, 2% magnesium stearate, and 1% surfactant | | | | | | |
| Surfactant | % Dissolved vs. Time (minutes) | | | | Minutes to dissolve: | |
| | 5 | 15 | 30 | 45 | 50% | 80% |
| No surfactant* | 28% | 57% | 79% | 95% | 11.8 | 30.7 |
| Polysorbate 80 | 38% | 74% | 96% | 100% | 7.3 | 17.8 |
| Sodium lauryl sulfate | 36% | 69% | 91% | 99% | 8.5 | 20.7 |
| Poloxamer 407 | 28% | 58% | 81% | 97% | 11.5 | 29.5 |
| Poloxamer 188 | 37% | 68% | 93% | 100% | 8.3 | 21.6 |
| Docusate sodium | 37% | 75% | 97% | 100% | 7.7 | 17.4 |

| | | | | | | |
|---|---|---|---|---|---|---|
| *Note: "No surfactant" case is 96% SODIUM OXYBATE instead of 95% SODIUM OXYBATE | | | | | | |

### Example 9- Lubricant Type

A 15-gram batch of alcohol granulation containing 98% sodium oxybate and 2% PVP K30 was made using procedures described in Example 7. Aliquots of the granulation were then blended with three lubricants at 2% levels - magnesium stearate, stearic acid powder, and sodium stearyl fumarate (Pruv®, JRS Pharma). Four tablets of 783 mg weight (0.3266" x 0.7283" oblong) were pressed, and hardness and dissolution were tested with 2 tablets. The results shown in Table 9, along with the "no lubricant" case from Example 7, indicate that sodium stearyl fumarate and stearic acid exhibit only a slight effect on dissolution.

**Table 9**

| Effect of Lubricant on Hardness and Dissolution of Sodium Oxybate Tablets | | | | | | | |
|---|---|---|---|---|---|---|---|
| Tablets contain 96% SODIUM OXYBATE, 2% PVP K30, and 2% lubricant | | | | | | | |
| Lubricant (2% level) | Tablet Hardness (N) | % Dissolved vs. Time (minutes) | | | | Minutes dissolve: | |
| | | 5 | 15 | 30 | 45 | 50% | 80% |
| No lubricant* | -- | 80% | 101% | 100% | 101% | 2.9 | 5.0 |
| Stearic acid | 119 | 61% | 96% | 100% | 100% | 4.1 | 9.1 |
| Sodium stearyl fumarate | 119 | 51% | 93% | 100% | 100% | 4.9 | 10.2 |
| Magnesium stearate | 102 | 27% | 54% | 76% | 91% | 12.9 | 33.3 |

| | | | | | | | |
|---|---|---|---|---|---|---|---|
| *Note: "No lubricant" case from Example 7 is 98% SODIUM OXYBATE and 2% PVP K30. | | | | | | | |

### Example 10- Other Tablet Strengths

The remaining granulation from Example 9 was blended with 2% sodium stearyl fumarate, and compressed into tablets of different size and shape. In all cases, 1-ton compression force and about 4-seconds dwell was used. The dissolution results shown in Table 10 confirm that tablets of 375 mg to 1500 mg strength perform comparably, with minor differences.

**Table 10**

| Dissolution Performance of Other Tablet Strengths | | | | | | | | |
|---|---|---|---|---|---|---|---|---|
| Strength (mg) | Mass (mg) | Tooling | Number Tested | % Dissolved vs. Time (minutes) | | | Minutes to dissolve: | |
| | | | | 5 | 15 | 30 | 50% | 80% |
| | | 3/8" | | | | | | |
| 375 | 392 | round | 2 | 47% | 95% | 100% | 5.4 | 9.7 |
| 752 | 783 | Oblong | 2 | 51% | 93% | 100% | 4.9 | 10.2 |
| 1000 | 1042 | Oblong | 1 | 43% | 91% | 101% | 6.1 | 11.6 |
| 1500 | 1562 | Oblong | 1 | 42% | 85% | 100% | 6.4 | 13.3 |

### Reference Example 11- Calcium Oxybate Immediate Release Tablet

Calcium oxybate was prepared by generally following procedures of Example 1 found in US 4,393,296 (Klosa, Production of Nonhygroscopic Salts of 4-Hydroxybutyric Acid). A small batch of granulation was made by first milling 8.35 grams of calcium oxybate to powder, then adding 1.66 grams of binder solution containing 10% PVP K30 in denatured alcohol. After hand mixing, granules were sized through a 16-mesh screen, and then dried for about 1 hour at 60ºC. Very hard granules were made, so gentle grinding with a mortar and pestle was required in order for all dried granules to pass through a 16-mesh screen. Finally, sodium stearyl fumarate was blended in at a 2% level.

Four tablets of 783 mg weight were made using 0.3266" x 0.7283" oblong tooling and a Carver press operated at 1-ton force and about 4-seconds dwell time. Two tablets were dissolution tested. Table 11 shows a comparison of the results between sodium oxybate and calcium oxybate prepared with otherwise the same formulation and methods.

**Table 11**

| Dissolution Results of Calcium Oxybate vs. Sodium Oxybate Tablets | | | | | | |
|---|---|---|---|---|---|---|
| API | Tablet Hardness (N) | % Dissolved vs. Time (minutes) | | | Minutes to dissolve: | |
| | | 5 | 15 | 30 | 50% | 80% |
| Calcium Oxybate | 151 | 45% | 86% | 100% | 6.0 | 13.0 |
| Sodium Oxybate | 119 | 51% | 93% | 100% | 4.9 | 10.2 |

### Example 12- Alcohol-granulated formulations

A 20-kg batch was made according to the formula in Table 12A using conditions summarized in Table 12B. The hydroxypropyl cellulose (HPC, Klucel EXF) was dissolved in 1800 g of ethanol to prepare the granulating solution. Sodium oxybate was screened through a 6 mesh Comil screen at very low RPM, and the remaining amount of HPC and sodium lauryl sulfate (SLS) were screened through a 20 mesh handscreen. The API, HPC and SLS were charged to the granulator bowl of a 150L TK-Fielder high-shear granulator, and were dry mixed for 5 minutes. The chopper was then turned on and the granulating solution was added over 3 minutes. The materials were mixed for another 5 minutes, then dried in a fluid bed dryer to a final LOD of 0.145%.

The dry granules were milled through a comill equipped with a 14 mesh screen at 1800rpm. Milled granules were mixed in a 2 cu ft V-blender for 5 minutes, then Pruv (previously screened through 30 mesh handscreen) was charged to the 2 cu ft V-blender and mixed for 3 minutes. The final blend was compressed at a target weight of 790 mg and hardness of 10.5 kp using a Kikusui 36 stations tablet press fitted with 0.329" x 0.712" oblong B-type tooling. The dissolution results by USP 2 (37°C, 50 rpm paddles, de-ionized water) using HPLC analysis indicated 35.3% dissolved at 5 minutes, 78.5% at 15 minutes, and complete dissolution in 30 minutes.

**Table 12A**

| Scaled-up Formulation using Alconol Granulation with HPC Binder | | | | |
|---|---|---|---|---|
| **Sodium Oxybate tablet** | | | | |
| **Ingredients** | | **% w/w** | **mg/tablet** | **Actua**l **kg/batch** |
| 1. | Sodium Oxybate | 95.00 | 750.00 | 19.0 |
| 2A. | Hydroxypropyl cellulose, NF ( Klucel EXF) in solution | 1.00 | 7.90 | 0.20 |
| 2B. | Hydroxypropyl cellulose, NF ( Klucel EXF) in the blend | 1.00 | 7.90 | 0.20 |
| 3. | Sodium Lauryl Sulfate, NF | 1.00 | 7.90 | 0.20 |
| 4. | Sodium Stearyl Fumarate, NF (Pruv) | 2.00 | 15.80 | 0.40 |
| 5. | Ethanol, USP | | | 1.80* |
| | **Total** | **100.0** | **789.50** | **20.00** |

| | | | | |
|---|---|---|---|---|
| * Removed during processing therefore not in the batch total. | | | | |

**Table 12B**

| Granulation, Drying, Milling, Compression Parameters | |
|---|---|
| **Wet granulation** | |
| Granulation solution addition rate | 600 g/min |
| Extra amount of ethanol added | none |
| Total granulation time (include solution | 3 minutes granulating solution |
| addition and wet mass) | 5 minutes wet mass |
| Impeller speed | 1800 |
| Chopper speed | 165rpm |

| **Fluid Bed Drying** | |
|---|---|
| Inlet drying temperature | 70-74°C |
| Exhaust temperature | 38-43°C |
| Drying time | 10 min |
| LOD_{final} | 0.145% |
| Air flow | 700-1000 cfm |

| **Milling** | |
|---|---|
| Quadro comil screen | 14 mesh |
| Impeller speed | 1800rpm |

| **Compression** | |
|---|---|
| Compression speed | 25rpm |

**Table 12C**

| Granulation Size Distribution | | | |
|---|---|---|---|
| Screen size US Std mesh | Opening size microns | Unmilled granules % Retained | Milled granules % Retained |
| 40 | 425 | 28.2 | 5.2 |
| 60 | 250 | 20.0 | 13.7 |
| 80 | 180 | 40.9 | 53.8 |
| 120 | 125 | 7.0 | 12.8 |
| 200 | 75 | 3.7 | 11.6 |
| 325 | 45 | 0.1 | 1.9 |
| Pan | <45 | 0.0 | 1.0 |
| Total | | 100 | 100 |

### Example 13- Formulation with Polyvinylpyrrolidone Binder

A formulation was demonstrated with a 20-kg batch using procedures comparable to those of Example 12. The formulation consisted of 96.25% sodium oxybate, 2.0% povidone K-30, and 1.75% sodium stearyl fumarate. The final blend was compressed at a target weight of 773mg and hardness of 11-13kp using a Kikusui 36 stations tablet press fitted with 0.329" x 0.712" oblong B-type tooling. The dissolution results by USP 2 (37ºC, 50 rpm paddles, de-ionized water) using HPLC analysis indicated 33.4% dissolved at 5 minutes, 77.7% at 15 minutes, and complete dissolution in 30 minutes.

## Claims

1. A compressed tablet immediate release formulation for oral delivery of sodium salt of gamma hydroxybutyrate (GHB), the compressed tablet immediate release formulation comprising:
sodium oxybate in an amount of 96-98% by weight of the immediate release formulation;
at least one binder selected from hypromellose, hydroxypropyl cellulose, sodium carboxymethylcellulose, polyvinyl alcohol, povidone, or starch in an amount of 1-2% by weight; and
at least one lubricant selected from magnesium stearate, stearic acid, and sodium stearyl fumarate in an amount of 1-2% by weight;
wherein the compressed tablet immediate release formulation comprises up to 2,000 mg of sodium oxybate and releases at least 90% of the sodium oxybate contained therein within a period of less than one hour after administration.

2. A compressed tablet immediate release formulation for oral delivery of sodium salt of gamma hydroxybutyrate (GHB), the compressed tablet immediate release formulation comprising:
sodium oxybate in an amount of 95-97.5% by weight of the immediate release formulation;
at least one binder selected from hypromellose, hydroxypropyl cellulose, sodium carboxymethylcellulose, polyvinyl alcohol, povidone, and starch in an amount of 1-2% by weight;
at least one lubricant selected from magnesium stearate, stearic acid, and sodium stearyl fumarate in an amount of 1-2% by weight; and
at least one surfactant selected from polysorbate 80, sodium lauryl sulfate, poloxamer, and docusate sodium in an amount of 0.5-1 % by weight;
wherein the compressed tablet immediate release formulation comprises up to 2,000 mg of sodium oxybate and releases at least 90% of the sodium oxybate contained therein within a period of less than one hour after administration.

3. The compressed tablet immediate release formulation of claim 1, wherein:
sodium oxybate is in an amount of 96% by weight;
the binder is hypromellose, hydroxypropyl cellulose, polyvinyl alcohol, or povidone in an amount of 2% by weight; and
the lubricant is magnesium stearate in an amount of 2% by weight.

4. The compressed tablet immediate release formulation of claim 3, wherein the binder is hydroxypropyl cellulose.

5. The compressed tablet immediate release formulation of claim 3, wherein the binder is povidone.

6. The compressed tablet immediate release formulation of claim 1, wherein:
sodium oxybate is in an amount of 96% by weight;
the binder is povidone in an amount of 2% by weight; and
the lubricant is stearic acid or sodium stearyl fumarate in an amount of 2% by weight.

7. The compressed tablet immediate release formulation of claim 1, wherein:
sodium oxybate is in an amount of 96.25% by weight;
the binder is povidone in an amount of 2% by weight; and
the lubricant is sodium stearyl fumarate in an amount of 1.75% by weight.

8. The compressed tablet immediate release formulation of claim 1, wherein:
sodium oxybate is in an amount of 96-97% by weight;
the binder is povidone in an amount of 2% by weight; and
the lubricant is magnesium stearate in an amount of 1-2% by weight.

9. The compressed tablet immediate release formulation of claim 2, wherein:
sodium oxybate is in an amount of 95% by weight;
the binder is povidone in amount of 2% by weight;
the lubricant is magnesium stearate in an amount of 2% by weight; and
the surfactant is selected from polysorbate 80, sodium lauryl sulfate, and poloxamer, docusate sodium in an amount of 1% by weight.

10. The compressed tablet immediate release formulation of claim 2, wherein:
sodium oxybate is in an amount of 95% by weight;
the binder is hydroxypropyl cellulose in an amount of 2% by weight;
the lubricant is sodium stearyl fumarate in an amount of 2% by weight; and
the surfactant is sodium lauryl sulfate in an amount of 1% by weight.

11. The compressed tablet immediate release formulation of claim 1 or 2, wherein the formulation comprises at least 500 mg of sodium oxybate.

12. The compressed tablet immediate release formulation of claim 1 or 2, wherein the formulation comprises at least 750 mg of sodium oxybate.

13. The compressed tablet immediate release formulation of claim 11, wherein the formulation comprises between about 500 mg and about about 1,500 mg of sodium oxybate.

14. The compressed tablet immediate release formulation of any of claims 1 to 13 for use in treatment of cataplexy and daytime sleepiness associated with narcolepsy.

15. The compressed tablet immediate release formulation of any of claims 1 to 13 for use in treatment of a condition selected from at least one of a movement disorder, restless leg syndrome, essential tremor, fibromyalgia, and chronic fatigue syndrome.

## Patentansprüche

1. Formulierung einer gepressten Tablette mit sofortiger Freisetzung für die orale Abgabe von Natriumsalz von Gamma-Hydroxybutyrat (GHB), wobei die Formulierung einer gepressten Tablette mit sofortiger Freisetzung Folgendes umfasst:
Natriumoxybat in einer Menge von 96-98 Gewichts-% der Formulierung mit sofortiger Freisetzung;
mindestens ein Bindemittel, das aus Hypromellose, Hydroxypropylcellulose, Natriumcarboxymethylcellulose, Polyvinylalkohol, Povidon oder Stärke ausgewählt ist, in einer Menge von 1-2 Gewichts-%; und
mindestens ein Schmiermittel, das aus Magnesiumstearat, Stearinsäure und Natriumstearylfumarat ausgewählt ist, in einer Menge von 1-2 Gewichts-%;
wobei die Formulierung einer gepressten Tablette mit sofortiger Freisetzung bis zu 2 000 mg Natriumoxybat umfasst und mindestens 90 % des Natriumoxybats, das darin enthalten ist, innerhalb einer Zeitdauer von weniger als einer Stunde nach der Verabreichung freisetzt.

2. Formulierung einer gepressten Tablette mit sofortiger Freisetzung für die orale Abgabe von Natriumsalz von Gamma-Hydroxybutyrat (GHB), wobei die Formulierung einer gepressten Tablette mit sofortiger Freisetzung Folgendes umfasst:
Natriumoxybat in einer Menge von 95-97,5 Gewichts-% der Formulierung mit sofortiger Freisetzung;
mindestens ein Bindemittel, das aus Hypromellose, Hydroxypropylcellulose, Natriumcarboxymethylcellulose, Polyvinylalkohol, Povidon oder Stärke ausgewählt ist, in einer Menge von 1-2 Gewichts-%;
mindestens ein Schmiermittel, das aus Magnesiumstearat, Stearinsäure und Natriumstearylfumarat ausgewählt ist, in einer Menge von 1-2 Gewichts-%; und
mindestens ein Tensid, das aus Polysorbat 80, Natriumlaurylsulfat, Poloxamer und Docusat-Natrium ausgewählt ist, in einer Menge von 0,5 bis 1 Gewichts-%;
wobei die Formulierung einer gepressten Tablette mit sofortiger Freisetzung bis zu 2 000 mg Natriumoxybat umfasst und mindestens 90 % des Natriumoxybats, das darin enthalten ist, innerhalb einer Zeitdauer von weniger als einer Stunde nach der Verabreichung freisetzt.

3. Formulierung einer gepressten Tablette mit sofortiger Freisetzung nach Anspruch 1, wobei:
Natriumoxybat in einer Menge von 96 Gewichts-% vorliegt;
das Bindemittel Hypromellose, Hydroxypropylcellulose, Polyvinylalkohol oder Povidon in einer Menge von 2 Gewichts-% ist; und
das Schmiermittel Magnesiumstearat in einer Menge von 2 Gewichts-% ist.

4. Formulierung einer gepressten Tablette mit sofortiger Freisetzung nach Anspruch 3, wobei das Bindemittel Hydroxypropylcellulose ist.

5. Formulierung einer gepressten Tablette mit sofortiger Freisetzung nach Anspruch 3, wobei das Bindemittel Povidon ist.

6. Formulierung einer gepressten Tablette mit sofortiger Freisetzung nach Anspruch 1, wobei:
Natriumoxybat in einer Menge von 96 Gewichts-% vorliegt;
das Bindemittel Povidon in einer Menge von 2 Gewichts-% ist; und
das Schmiermittel Stearinsäure oder Natriumstearylfumarat in einer Menge von 2 Gewichts-% ist.

7. Formulierung einer gepressten Tablette mit sofortiger Freisetzung nach Anspruch 1, wobei:
Natriumoxybat in einer Menge von 96,25 Gewichts-% vorliegt;
das Bindemittel Povidon in einer Menge von 2 Gewichts-% ist; und
das Schmiermittel Natriumstearylfumarat in einer Menge von 1,75 Gewichts-% ist.

8. Formulierung einer gepressten Tablette mit sofortiger Freisetzung nach Anspruch 1, wobei:
Natriumoxybat in einer Menge von 96-97 Gewichts-% vorliegt;
das Bindemittel Povidon in einer Menge von 2 Gewichts-% ist; und
das Schmiermittel Magnesiumstearat in einer Menge von 1-2 Gewichts-% ist.

9. Formulierung einer gepressten Tablette mit sofortiger Freisetzung nach Anspruch 2, wobei:
Natriumoxybat in einer Menge von 95 Gewichts-% vorliegt;
das Bindemittel Povidon in einer Menge von 2 Gewichts-% ist;
das Schmiermittel Magnesiumstearat in einer Menge von 2 Gewichts-% ist; und
das Tensid aus Polysorbat 80, Natriumlaurylsulfat und Poloxamer, Docusat-Natrium in einer Menge von 1 Gewichts-% ausgewählt ist.

10. Formulierung einer gepressten Tablette mit sofortiger Freisetzung nach Anspruch 2, wobei:
Natriumoxybat in einer Menge von 95 Gewichts-% vorliegt;
das Bindemittel Hydroxypropylcellulose in einer Menge von 2 Gewichts-% ist;
das Schmiermittel Natriumstearylfumarat in einer Menge von 2 Gewichts-% ist; und
das Tensid Natriumlaurylsulfat in einer Menge von 1 Gewichts-% ist.

11. Formulierung einer gepressten Tablette mit sofortiger Freisetzung nach Anspruch 1 oder 2, wobei die Formulierung mindestens 500 mg Natriumoxybat umfasst.

12. Formulierung einer gepressten Tablette mit sofortiger Freisetzung nach Anspruch 1 oder 2, wobei die Formulierung mindestens 750 mg Natriumoxybat umfasst.

13. Formulierung einer gepressten Tablette mit sofortiger Freisetzung nach Anspruch 11, wobei die Formulierung zwischen etwa 500 mg und etwa 1 500 mg Natriumoxybat umfasst.

14. Formulierung einer gepressten Tablette mit sofortiger Freisetzung nach einem der Ansprüche 1 bis 13 für die Verwendung bei der Behandlung von Kataplexie und Tagesmüdigkeit, die mit Narkolepsie verbunden sind.

15. Formulierung einer gepressten Tablette mit sofortiger Freisetzung nach einem der Ansprüche 1 bis 13 für die Verwendung bei der Behandlung eines Leidens, das aus mindestens einem von Folgenden ausgewählt ist: einer Bewegungsstörung, Syndrom der unruhigen Beine, essenziellem Tremor, Fibromyalgie und chronischem Müdigkeitssyndrom.

## Revendications

1. Formulation à libération immédiate en comprimé destinée à une administration orale d'un sel de sodium de gamma-hydroxybutyrate (GHB), la formulation à libération immédiate en comprimé comprenant :
de l'oxybate de sodium selon une quantité de 96-98% en poids de la formulation à libération immédiate ;
au moins un liant choisi parmi l'hypromellose, l'hydroxypropyl cellulose, la carboxyméthylcellulose de sodium, l'alcool polyvinylique, la povidone, ou l'amidon, selon une quantité de 1-2% en poids ; et
au moins un lubrifiant choisi parmi le stéarate de magnésium, l'acide stéarique, et le fumarate de stéaryle sodique, selon une quantité de 1-2% en poids ;
la formulation à libération immédiate en comprimé comprenant jusqu'à 2000 mg d'oxybate de sodium et libérant au moins 90% de l'oxybate de sodium y étant contenu au sein d'une période inférieure à une heure après administration.

2. Formulation à libération immédiate en comprimé destinée à une administration orale d'un sel de sodium de gamma-hydroxybutyrate (GHB), la formulation à libération immédiate en comprimé comprenant :
de l'oxybate de sodium selon une quantité de 95-97,5% en poids de la formulation à libération immédiate ;
au moins un liant choisi parmi l'hypromellose, l'hydroxypropyl cellulose, la carboxyméthylcellulose de sodium, l'alcool polyvinylique, la povidone, et l'amidon, selon une quantité de 1-2% en poids ;
au moins un lubrifiant choisi parmi le stéarate de magnésium, l'acide stéarique, et le fumarate de stéaryle sodique, selon une quantité de 1-2% en poids ; et
au moins un agent tensioactif choisi parmi le polysorbate 80, le laurylsulfate de sodium, un poloxamère, et le docusate de sodium, selon une quantité de 0,5-1% en poids ;
la formulation à libération immédiate en comprimé comprenant jusqu'à 2000 mg d'oxybate de sodium et libérant au moins 90% de l'oxybate de sodium y étant contenu au sein d'une période inférieure à une heure après administration.

3. Formulation à libération immédiate en comprimé selon la revendication 1, dans laquelle :
l'oxybate de sodium est présent selon une quantité de 96% en poids ;
le liant est constitué d'hypromellose, d'hydroxypropyl cellulose, d'alcool polyvinylique, ou de povidone, selon une quantité de 2% en poids ; et
le lubrifiant est constitué de stéarate de magnésium, selon une quantité de 2% en poids.

4. Formulation à libération immédiate en comprimé selon la revendication 3, dans laquelle le liant est constitué d'hydroxypropyl cellulose.

5. Formulation à libération immédiate en comprimé selon la revendication 3, dans laquelle le liant est constitué de povidone.

6. Formulation à libération immédiate en comprimé selon la revendication 1, dans laquelle :
l'oxybate de sodium est présent selon une quantité de 96% en poids ;
le liant est constitué de povidone, selon une quantité de 2% en poids ; et
le lubrifiant est constitué d'acide stéarique ou de fumarate de stéaryle sodique, selon une quantité de 2% en poids.

7. Formulation à libération immédiate en comprimé selon la revendication 1, dans laquelle :
l'oxybate de sodium est présent selon une quantité de 96,25% en poids ;
le liant est constitué de povidone, selon une quantité de 2% en poids ; et
le lubrifiant est constitué de fumarate de stéaryle sodique, selon une quantité de 1,75% en poids.

8. Formulation à libération immédiate en comprimé selon la revendication 1, dans laquelle :
l'oxybate de sodium est présent selon une quantité de 96-97% en poids ;
le liant est constitué de povidone, selon une quantité de 2% en poids ; et
le lubrifiant est constitué de stéarate de magnésium, selon une quantité de 1-2% en poids.

9. Formulation à libération immédiate en comprimé selon la revendication 2, dans laquelle :
l'oxybate de sodium est présent selon une quantité de 95% en poids ;
le liant est constitué de povidone, selon une quantité de 2% en poids ;
le lubrifiant est constitué de stéarate de magnésium, selon une quantité de 2% en poids ; et
l'agent tensioactif est choisi parmi le polysorbate 80, le laurylsulfate de sodium, un poloxamère, et le docusate de sodium, selon une quantité de 1% en poids.

10. Formulation à libération immédiate en comprimé selon la revendication 2, dans laquelle :
l'oxybate de sodium est présent selon une quantité de 95% en poids ;
le liant est constitué d'hydroxypropyl cellulose, selon une quantité de 2% en poids ;
le lubrifiant est constitué de fumarate de stéaryle sodique, selon une quantité de 2% en poids ; et
l'agent tensioactif est constitué de laurylsulfate de sodium, selon une quantité de 1% en poids.

11. Formulation à libération immédiate en comprimé selon la revendication 1 ou 2, la formulation comprenant au moins 500 mg d'oxybate de sodium.

12. Formulation à libération immédiate en comprimé selon la revendication 1 ou 2, la formulation comprenant au moins 750 mg d'oxybate de sodium.

13. Formulation à libération immédiate en comprimé selon la revendication 11, la formulation comprenant entre environ 500 mg et environ 1500 mg d'oxybate de sodium.

14. Formulation à libération immédiate en comprimé selon l'une quelconque des revendications 1 à 13, destinée à une utilisation dans le traitement de la cataplexie et de la somnolence diurne associée à la narcolepsie.

15. Formulation à libération immédiate en comprimé selon l'une quelconque des revendications 1 à 13, destinée à une utilisation dans le traitement d'une condition choisie parmi au moins l'un parmi un trouble du mouvement, le syndrome des jambes sans repos, le tremblement essentiel, la fibromyalgie, et le syndrome de fatigue chronique.
